# EUROPÄISCHE PATENTSCHRIFT

(11) **EP 4 166 102 B1**
(45) Veröffentlichungstag und Bekanntmachung des Hinweises auf die Patenterteilung: **04.03.2026**
(21) Anmeldenummer: 21202393.1
(22) Anmeldetag: 13.10.2021
(51) Int. Cl.: A61B 17/64

(54) **KLEMMVORRICHTUNG FÜR EINEN EXTERNEN FIXATEUR**
CLAMPING DEVICE FOR AN EXTERNAL FIXATOR
DISPOSITIF DE SERRAGE POUR UN FIXATEUR EXTERNE

(43) Veröffentlichungstag der Anmeldung: 19.04.2023
(73) Patentinhaber: Schiffers, Hank, 47259 Duisburg (DE)
(72) Erfinder: Schiffers, Hank, 47259 Duisburg (DE)
(74) Vertreter: Kluin Patent

(56) Entgegenhaltungen:
- EP-A1- 2 250 968
- US-A- 4 653 481
- US-A1- 2012 004 659
- US-A1- 2018 132 897

## Beschreibung

Die Erfindung betrifft eine Klemmvorrichtung zum Verbinden von Pins und/oder Stabelementen eines externen Fixateurs zur Behandlung von Knochenbrüchen. Die Klemmvorrichtung weist eine erste Klemme und eine zweite Klemme auf, wobei entlang einer axialen Richtung in einer durchgehenden Bohrung der ersten Klemme und der zweiten Klemme ein, die erste Klemme mit der zweiten Klemme verbindendes, Zugelement vorgesehen ist. Das Zugelement ist mit einem ersten Ende in der zweiten Klemme gelagert und weist ein, dem ersten Ende gegenüberliegendes Gewinde auf, welches in ein Gegengewinde eines Anziehmittels eingreift. Das Zugelement ist zum Verspannen der ersten Klemme und der zweiten Klemme durch Einschrauben des Gewindes in das Gegengewinde vorgesehen. Die Klemmvorrichtung weist weiterhin ein Federmittel zum Vorspannen der ersten Klemme und der zweiten Klemme auf.

Solche Klemmen zur Verwendung in einem externen Fixateur sind aus dem Stand der Technik bekannt. Ein externer Fixateur dient der Stabilisierung und der Fixierung von Knochenfragmenten zur notfallmäßigen, vorläufigen Behandlung eines Knochenbruchs und weist typischerweise eine Mehrzahl von stangenförmigen Pins, Klemmvorrichtungen und mindestens ein Stabelement auf.

Die Pins, welche üblicherweise einen deutlich geringeren Durchmesser aufweisen als die Stabelemente, sind dazu vorgesehen, in den Knochenfragmente so verankert zu werden, dass die Pins teilweise durch die Haut aus dem Körper der verletzten Person herausragen. Hierzu sind die Pins beispielsweise in Form von Schrauben ausgebildet, welche durch die Haut der verletzten Person in die Knochenfragmente eingeschraubt werden.

Die aus der Haut herausragenden Enden der Pins werden mit Klemmvorrichtungen über die Stabelemente miteinander verbunden und relativ zueinander fixiert. Bei einfachen Knochenbrüchen kann es ausreichend sein, dass lediglich zwei Knochenfragmente mit Pins versehen und die Pins mit nur einem Stab miteinander verbunden werden. Liegt eine kompliziertere Knochenfraktur vor, so ist es mitunter notwendig, dass viele Knochenfragmente miteinander verbunden werden müssen. Hierzu wird eine Vielzahl von Pins in die Knochenfragmente eingeschraubt und über mehrere Stabelemente, wenn nötig auch über Gelenke hinweg, miteinander verbunden.

Ein Vorteil der Stabilisierung einer Knochenfraktur mithilfe eines externen Fixateurs ist, dass die Knochenfragmente nicht freigelegt werden müssen. Mithilfe des externen Fixateurs kann eine provisorische Stabilisierung der Knochenfragmente erfolgen, sodass die verletzte Person transportfähig ist. Liegen weitere Verletzungen, insbesondere lebensbedrohliche Verletzungen vor, erlaubt die Stabilisierung der Knochenfraktur mit dem externen Fixateur, bei provisorisch stabilisierter Knochenfraktur zunächst die weiteren Verletzungen zu behandeln.

Besonders vorteilhaft ist die Verwendung eines externen Fixateurs nach Naturkatastrophen oder in Kriegsgebieten. So ist es möglich, verletzte Personen rasch mit stabilisierten Knochenfrakturen aus dem Katastrophengebiet bzw. der Gefechtszone zu transportieren. Ist der Patient in einem Zustand, welcher es zulässt, dass der Knochenbruch operiert werden kann, so wird der externe Fixateur entnommen und die Knochenfragmente mit internen Hilfsmitteln, beispielsweise mit Nägeln, Platten oder Schrauben, miteinander fixiert und stabilisiert.

Von besonderer Wichtigkeit ist bei der Verwendung eines externen Fixateurs die einfache Bedienbarkeit der Klemmvorrichtungen. Gerade in Stresssituationen und bei einer besonderen Eiligkeit der Versorgung der verletzten Person ist es wünschenswert, dass die Klemmvorrichtungen ohne Werkzeug bedienbar und mit nur wenigen Handgriffen angelegt und fixiert werden können.

Aus der Druckschrift US 2012 /089 142 A1 ist eine Klemmvorrichtung bekannt, welche zwei Klemmen aufweist. Beide Klemmen verbindet ein Bolzen, welcher an seinen beiden Enden jeweils ein Gewinde aufweist. Die Gewinde greifen in Muttern ein. Werden die Muttern weiter auf das Gewinde des Bolzens geschraubt, so werden die Klemmen verspannt und in ihrer Position fixiert. Zwei Federn dienen der Vorspannung der beiden Klemmen, um von einer weit geöffneten Position in einen provisorisch verschlossenen Zustand überzugehen. Die Druckschrift US 2018 / 132 897 A1 offenbart eine Klemmvorrichtung zum Verbinden von Pins und Stabelementen eines externen Fixateurs. Die Klemmvorrichtung weist eine erste Klemme und eine zweite Klemme, wobei entlang einer axialen Richtung in einer durchgehenden Bohrung der ersten Klemme (10) und der zweiten Klemme ein, die erste Klemme mit der zweiten Klemme verbindendes, Zugelement vorgesehen ist. Das Zugelement ist mit einem ersten Ende in der zweiten Klemme gelagert und weist an einem gegenüberliegenden zweiten Ende, ein Gewinde auf. Das Gewinde greift, das in ein Gegengewinde eines Anziehmittels ein, wobei das Zugelement zum Verspannen der ersten Klemme und der zweiten Klemme durch Einschrauben des Gewindes in das Gegengewinde vorgesehen ist. Ferner weist die Klemmvorrichtung ein Federmittel zum Vorspannen der ersten Klemme und der zweiten Klemme auf.

Aufgabe der vorliegenden Erfindung ist es, eine alternative Klemmvorrichtung bereitzustellen, welche sehr einfach bedienbar ist und mit einer im Vergleich zum Stand der Technik deutlich reduzierten Anzahl von Komponenten auskommt.

Die Aufgabe der Erfindung wird gelöst durch eine Klemmvorrichtung nach Anspruch 1.

Bei der erfindungsgemäßen Klemmvorrichtung ist das Anziehmittel zwischen dem Federmittel und dem Zugelement angeordnet. Im Sinne der vorliegenden Erfindung bezieht sich "zwischen" auf die axiale Richtung. Das Zugelement ist mit dem ersten Ende in der zweiten Klemme gelagert. Dem ersten Ende liegt in axialer Richtung das zweite Ende gegenüber, an welchem das Gewinde des Zugelementes angeordnet ist. Vorzugsweise ist vorgesehen, dass die erste Klemme und zumindest teilweise die zweite Klemme zwischen dem ersten Ende und dem zweiten Ende des Zugelementes angeordnet sind. Weiterhin ist vorzugsweise vorgesehen, dass das Anziehmittel direkt oder indirekt an der ersten Klemme anliegend angeordnet ist. Insbesondere ist vorgesehen, dass das Anziehmittel indirekt einer ersten Klemme anliegend angeordnet ist, d. h., dass das Anziehmittel durch ein weiteres Bauteil mit der ersten Klemme in Wirkverbindung steht. Wird das Gewinde des Zugelementes in das Gegengewinde des Anziehmittels eingeschraubt, so werden die erste Klemme und die zweite Klemme zusammengedrückt. Das Zusammendrücken der Klemmen bewirkt eine Verspannung der Klemmen, wodurch Stabelemente bzw. Pins in den Klemmen gehalten werden.

Sind die Klemmen der Klemmvorrichtung nicht verspannt, so bewirkt das Federmittel der Klemmvorrichtung eine Vorspannung. Durch die Vorspannung ist es möglich, dass Stabelemente bzw. Pins durch die Klemmen gehalten werden können, ohne dass die Klemmen fest verspannt sind. Dies ermöglicht auf vorteilhafte Weise eine einfache Bedienung und Justage der Klemmvorrichtung. Das Zugelement, das Anziehmittel und das Federmittel sind relativ zueinander so angeordnet, dass, bezogen auf die axiale Richtung, das Anziehmittel zwischen dem Federmittel und dem Zugelement angeordnet ist. Hierdurch ergeben sich deutlich vorteilhaftere Kraftflüsse beim Vorspannen und Verspannen der Klemmen, sodass die maximale Lebensdauer der Klemmvorrichtung vorteilhaft verlängert wird.

Vorzugsweise ist vorgesehen, dass die erste Klemme, die zweite Klemme, das Zugelement und dass Anziehmittel aus einem Metall hergestellt sind. Denkbar ist, dass das Zugelement ein zugbelastbares längliches Bauteil ist. Denkbar ist, dass das Zugelement eine Kette oder ein Seil aufweist. Denkbar ist aber auch, dass das Zugelement eine Pfostenkomponente aufweist.

Vorteilhafte Ausgestaltungen und Weiterbildungen der Erfindung sind den Unteransprüchen, sowie der Beschreibung unter Bezugnahme auf die Zeichnungen entnehmbar.

Gemäß einer bevorzugten Ausführungsform der vorliegenden Erfindung ist vorgesehen, dass die erste Klemme eine erste Klemmbacke und eine zweite Klemmbacke aufweist und dass die zweite Klemme eine dritte Klemmbacke und eine vierte Klemmbacke aufweist, wobei die erste Klemmbacke, die zweite Klemmbacke, die dritte Klemmbacke sowie die vierte Klemmbacke jeweils eine Nut aufweisen, wobei durch die Nuten der ersten Klemmbacke und der zweiten Klemmbacke eine offene erste Aufnahmenut zum Aufnehmen eines Stabelementes gebildet ist und durch die Nuten der dritten Klemmbacke und der vierten Klemmbacke eine offene zweite Aufnahmenut zur Aufnahme eines Stabelementes eines gebildet ist, wobei die dritte Klemmbacke und die vierte Klemmbacke jeweils eine, vorzugsweise zwei weiteren Nuten aufweisen, wobei durch die weiteren Nuten der dritten Klemmbacke und der vierten Klemmbacke mindestens eine, vorzugsweise zwei, offene weitere Aufnahmenuten zur Aufnahme eines Pins gebildet sind. Hierzu ist vorzugsweise vorgesehen, dass die erste Aufnahmenut zumindest abschnittsweise mit ersten Oberflächenstrukturierungen versehen ist, die dazu ausgebildet sind, in komplementär ausgebildete erste weitere Oberflächenstrukturierungen eines Stabelements formschlüssig einzugreifen und/oder die zweite Aufnahmenut zumindest abschnittsweise vorzugsweise mit zweiten Oberflächenstrukturierungen versehen ist, die dazu ausgebildet sind, in komplementär ausgebildete erste weitere Oberflächenstrukturierungen eines Stabelements formschlüssig einzugreifen und/oder die weitere Aufnahmenut bzw. die weiteren Aufnahmenuten zumindest abschnittsweise vorzugsweise mit dritten Oberflächenstrukturierungen versehen ist, die dazu ausgebildet sind, in komplementär ausgebildete zweite weitere Oberflächenstrukturierungen eines Pins formschlüssig einzugreifen.

Hierdurch ist auf vorteilhafte Weise bewirkt, dass Pins bzw. Stabelemente auch bei nicht verspannten Klemmen stabil gehalten werden können. Vorzugsweise ist vorgesehen, dass die erste Klemmbacke, die zweite Klemmbacke, die dritte Klemmbacke und/oder die vierte Klemmbacke einstückig ausgestaltet sind. Weiterhin ist vorzugsweise vorgesehen, dass die erste Klemmbacke, die zweite Klemmbacke, die dritte Klemmbacke und/oder die vierte Klemmbacke aus einem Metall, beispielsweise Aluminium, Stahl oder Titan, hergestellt sind. Vorzugsweise sind die Nuten der ersten Klemmbacke und der zweiten Klemmbacke entlang der axialen Richtung gegenüberliegend angeordnet. Weiterhin sind vorzugsweise die Nuten der zweiten Klemmbacke und der vierten Klemmbacke entlang der axialen Richtung gegenüberliegend angeordnet. Besonders bevorzugt ist vorgesehen, dass die weiteren Aufnahmenuten zueinander parallel verlaufend angeordnet sind. Weiterhin ist besonders bevorzugt vorgesehen, dass die weiteren Aufnahmenuten winkelig und insbesondere senkrecht zum Verlauf der zweiten Aufnahmenut verlaufend angeordnet sind.

Denkbar ist, dass die erste und zweite Klemmbacke ineinandergreifende Strukturen aufweisen, die dazu vorgesehen sind, eine Rotation der ersten Klemmbacke relativ zur zweiten Klemmbacke zu verhindern. Weiterhin ist denkbar, dass die dritte und vierte Klemmbacke ineinandergreifende Strukturen aufweisen, die dazu vorgesehen sind, eine Rotation der dritten Klemmbacke relativ zur vierten Klemmbacke zu verhindern. Hierzu ist denkbar, dass die ineinandergreifenden Strukturen entlang der axialen Richtung hervorstehende Stifte, welche in Vertiefungen eingreifen, aufweisen. Dies verhindert ein Verschieben der Nuten relativ zueinander. Ferner ist denkbar, dass die erste Oberflächenstrukturierungen, die zweite Oberflächenstrukturierungen und/oder die dritte Oberflächenstrukturierungen eine gezahnte oder geriffelte Struktur aufweisen.

Das Federmittel bewirkt eine Vorspannung der Gestalt, dass im vorgespannten Zustand der Klemmvorrichtung der Durchmesser der ersten Aufnahmenut kleiner ist als der Durchmesser eines Stabelementes. Weiterhin wird durch die Vorspannung des Federmittels bewirkt, dass im vorgespannten Zustand der Klemmvorrichtung der Durchmesser der zweiten Aufnahmenut geringer ist als der Durchmesser eines Stabelementes und der Durchmesser der weiteren Aufnahmenuten geringer ist als der Durchmesser eines Pins. Zum Einführen eines Stabelementes bzw. eines Pins in eine Aufnahmenut bzw. eine weitere Aufnahmenut ist vorgesehen, dass das Stabelement bzw. der Pin seitlich, d. h. mit einer Bewegungsrichtung, welche im Wesentlichen orthogonal zum Verlauf der jeweiligen Aufnahmenut bzw. weiteren Aufnahmenut angeordnet ist, in die Aufnahmenut bzw. weitere Aufnahmenut eingedrückt wird. Hierbei wird entgegen der Vorspannung der Durchmesser der Aufnahmenut bzw. der weiteren Aufnahmenut kurzzeitig vergrößert, bevor sich der Durchmesser bei in der Aufnahmenut bzw. weiteren Aufnahmenut angeordnetem Stabelement bzw. Pin wieder verkleinert. Der Durchmesser der Aufnahmenut entspricht bei in der Aufnahmenut angeordnetem Stabelement dem Durchmesser des Stabelementes. Der Durchmesser der weiteren Aufnahmenut entspricht bei in der weiteren Aufnahmenut angeordnetem Pin dem Durchmesser des Pins. Der Durchmesser im Sinne der vorliegenden Erfindung ist der maximale Abstand entlang der axialen Richtung zwischen den beiden Nuten einer Aufnahmenut bzw. einer weiteren Aufnahmenut.

Dadurch, dass lediglich seitlich offene Aufnahmenuten bzw. seitlich offene weitere Aufnahmenuten zur Aufnahme von Stabelementen bzw. Pins vorhanden sind, kann auf die Verwendung von Gehäusen, insbesondere auf die Verwendung von Gehäusen zur Aufnahme von Pins oder zur Aufnahme von Stabelementen auf vorteilhafte Weise verzichtet werden. Vorzugsweise weist daher die Klemmvorrichtung kein geschlossenes Gehäuse zur Aufnahme eines Stabelementes und/oder kein geschlossenes Gehäuse zur Aufnahme eines Pins auf.

Vorzugsweise ist vorgesehen, dass an der offenen Seite der Aufnahmenuten abgerundete Einführbereiche zur Erleichterung des Einführens der Stabelemente bzw. Pins angeordnet sind. Die abgerundeten Bereiche weisen keine geraden Flächen auf und ermöglichen einen deutlich einfacheren Zusammenbau des Fixateurs.

Erfindungsgemäß ist vorgesehen, dass die Klemmvorrichtung ein Handrad aufweist, wobei das Handrad zum Drehen des Anziehmittels mit dem Anziehmittel in Eingriff steht, wobei das Anziehmittel entlang der axialen Richtung an dem Handrad anliegend angeordnet ist. Hierdurch ist es auf vorteilhafte Weise möglich, mittels des Handrades das Anziehmittel zu drehen und so das Gewinde in das Gegengewinde einzuschrauben. Vorzugsweise ist das Handrad zu den restlichen Elementen der Klemmvorrichtung farblich abgesetzt. Hieraus ergibt sich der Vorteil, dass dem Bediener der Klemmvorrichtung selbst in einer Stresssituation sofort klar ist, an welcher Stelle die Klemmvorrichtung zu bedienen ist. Vorzugsweise ist weiterhin vorgesehen, dass das Handrad aus einem Metall gefertigt ist. Denkbar ist, dass das Handrad eine seitliche Strukturierung zur Erleichterung der Bedienung aufweist. Vorzugsweise ist vorgesehen, dass das Handrad relativ zur ersten Klemme drehbar ist. Weiterhin ist vorzugsweise vorgesehen, dass das Handrad an der ersten Klemme anliegend angeordnet ist. Insbesondere ist vorgesehen, dass die erste Klemme zwischen der zweiten Klemme und dem Handrad angeordnet ist. Gemäß einer bevorzugten Ausführungsform ist vorgesehen, dass das Handrad die erste Klemme teilweise einhüllend angeordnet ist. Hierzu ist vorzugsweise vorgesehen, dass das Handrad eine Auskragung aufweist, welche die erste Klemmbacke teilweise umgreift.

Vorzugsweise ist vorgesehen, dass das Handrad ein Abstützelement aufweist, welches an der ersten Klemmbacke anliegend angeordnet ist. Denkbar ist, dass das Abstützelement entlang der axialen Richtung auf der der ersten Klemmbacke zugewandten Seite des Handrades hervorsteht. Denkbar ist ferner, dass das Abstützelement ringförmig um das Anziehmittel herum angeordnet ist. Das Anziehmittel dient der sicheren Anlage des Handrades an der ersten Klemmbacke und überträgt die Federkraft des Federelementes zum Vorspannen vom Handrad auf die erste Klemmbacke.

Erfindungsgemäß ist vorgesehen, dass das Anziehmittel zwischen dem Handrad und der ersten Klemme angeordnet ist, wobei das Gegengewinde des Anziehmittels in einer mit der durchgehenden Bohrung fluchtenden ersten weiteren Bohrung des Anziehmittels angeordnet ist, wobei ein Eingriffteil des Anziehmittels formschlüssig in Eingriff mit einer Aufnahme des Handrades steht. Wird das Handrad gedreht, so bewirkt die Drehbewegung auf vorteilhafte Weise eine Bewegung des Zugelementes entlang der axialen Richtung. Insbesondere ist vorgesehen, dass das Handrad und das Anziehmittel zwei separate Bauteile sind.

Hierzu ist erfindungsgemäß vorgesehen, dass das Handrad eine Ausnehmung aufweist, wobei das Federmittel innerhalb der Ausnehmung angeordnet ist, wobei das Federmittel an einer Stützstelle des Handrades und an einem Vorspannmittel anliegend angeordnet ist, wobei das Vorspannmittel in Eingriff mit dem Zugelement steht, wobei das Federmittel so angeordnet ist, dass eine Federkraft des Federmittels das Zugelement entlang der axialen Richtung in Richtung des Handrades zieht. Hierdurch ist auf vorteilhafte Weise eine sehr geschickte Führung der Kräfte zur Vorspannung bewirkt. Vorzugsweise ist vorgesehen, dass das Federmittel so angeordnet ist, dass die Federkraft des Federmittels entlang der axialen Richtung angeordnet ist. Denkbar ist weiterhin, dass die Ausnehmung des Handrades mit der Bohrung fluchtend angeordnet ist. Vorzugsweise ist vorgesehen, dass das Federmittel vollständig in der Ausnehmung angeordnet ist.

Gemäß einer weiteren bevorzugten Ausführungsform der vorliegenden Erfindung ist vorgesehen, dass das Vorspannmittel eine Sicherungsschraube aufweist, deren weiteres Gewinde in Eingriff mit einem weiteren Gegengewinde des Zugelementes steht, wobei vorzugsweise das Federmittel an einer der Stützstelle zugewandten Unterseite eines Schraubenkopfes der Sicherungsschraube anliegend angeordnet ist. Das Vorspannmittel ist also dazu vorgesehen, die Federkraft des Federmittels auf das Zugelement so zu übertragen, dass das Zugelement in einem vorgespannten Zustand angeordnet ist. Hierzu sorgt das Federmittel in Verbindung mit dem Zugelement dafür, dass die Klemmbacken der Klemmen zwischen dem Lager des Zugelementes in der zweiten Klemmbacke und dem Handrad so zusammengedrückt werden, dass Stabelemente bzw. Pins in die entsprechenden Aufnahmenuten eingesteckt werden können und dort selbstständig stabil verbleiben. Vorzugsweise ist vorgesehen, dass der Schraubenkopf der Sicherungsschraube in axialer Richtung das Handrad nicht überragt. Die Sicherungsschraube sichert ferner die Klemmvorrichtung gegen unbeabsichtigtes Demontieren.

Zur Übertragung der Federkraft des Federmittels auf das Handrad ist vorzugsweise vorgesehen, dass die Stützstelle zwischen der Aufnahme des Handrades und der Ausnehmung des Handrades angeordnet ist, wobei die Stützstelle vorzugsweise einstückig mit dem Handrad ausgebildet ist oder wobei die Stützstelle eine ringförmige Scheibe aufweist, welche in eine Ringnut des Handrades eingesetzt ist. Vorzugsweise ist vorgesehen, dass die Sicherungsschraube die Stützstelle durchgreift. Hierzu ist vorgesehen, dass die Stützstelle eine Öffnung aufweist, welche von einem Teil der Sicherungsschraube durchgriffen wird. Insbesondere ist vorgesehen, dass der Schraubenkopf der Sicherungsschraube so bemessen ist, dass dieser nicht durch die Öffnung passt. Weiterhin ist vorzugsweise vorgesehen, dass das Federmittel um die Sicherungsschraube herum angeordnet ist.

Gemäß einer weiteren bevorzugten Ausführungsform der vorliegenden Erfindung ist vorgesehen, dass die Aufnahme einen profilierten Querschnitt, vorzugsweise einen wellenförmigen und/oder eckigen, besonders bevorzugt quadratischen, sechs- oder achteckigen Querschnitt aufweist, wobei der Eingriffteil an einem oberen Abschnitt des Anziehmittels angeordnet ist, wobei der obere Abschnitt einen profilierten Querschnitt, vorzugsweise einen wellenförmigen und/oder eckigen, besonders bevorzugt quadratischen oder sechseckigen, Querschnitt aufweist.. Hierdurch ist auf vorteilhafte Weise sichergestellt, dass das Handrad sicher mit dem Eingriffteil in Eingriff steht. Vorzugsweise ist hierzu vorgesehen, dass das Anziehmittel einen unteren Abschnitt aufweist, wobei der untere Abschnitt in der Bohrung angeordnet ist, wobei der untere Abschnitt vorzugsweise einen runden Querschnitt aufweist.

Zur Bereitstellung einer hohen Flexibilität der Klemmvorrichtung ist vorzugsweise vorgesehen, dass die erste Klemme und die zweite Klemme über ein Drehgelenk um eine Drehachse zueinander drehbar und über ein Schwenkgelenk um eine senkrecht zur Drehachse angeordnete Schwenkachse zueinander schwenkbar gelagert sind, wobei das Zugelement zum Fixieren der ersten Klemme relativ zur zweiten Klemme bezüglich der Drehachse und der Schwenkachse durch Einschrauben des Gewindes in das Gegengewinde vorgesehen ist. Hierdurch ist auf vorteilhafte Weise möglich, dass die Klemmvorrichtung gleichzeitig bezüglich der Drehachse und bezüglich der Schwenkachse fixiert wird und weiterhin die Klemmen verspannt werden. Für ein Verbringen der Klemmvorrichtung von dem vorgespannten Zustand, in welchem Stabelemente und Pins eingelegt und die Orientierungen der Klemmen bezüglich der Drehachse und der Schwenkachse eingestellt werden können, in den verspannten Zustand, in dem die Klemmvorrichtung Pins und Stabelemente sicher in einer vorgegebenen Stellung fixiert, ist somit lediglich das Aufbringen von Zug auf das Zugelement vorzugsweise in einem einzelnen Arbeitsschritt, nötig. Hierdurch wird die Handhabung der Klemmvorrichtung deutlich vereinfacht. Die Versorgung von verletzten Personen mit einem externen Fixateur wird gerade in Stresssituationen erleichtert und die zur Versorgung notwendige Zeit verkürzt.

Gemäß einer weiteren bevorzugten Ausführungsform der vorliegenden Erfindung ist vorgesehen, dass das Drehgelenk zumindest zwei zueinander um die Drehachse drehbar gelagerte und aneinander angrenzende Drehgelenkkomponenten umfasst, die mittels des Zugelements relativ zueinander in unterschiedlichen Winkelstellungen arretierbar sind, wobei zwischen den Drehgelenkkomponenten ein erstes weiteres Federmittel angeordnet ist, welches dazu ausgebildet ist, die Drehgelenckomponenten in axialer Richtung voneinander weg zu spannen, wobei die Drehgelenkkomponenten einander gegenüberliegend angeordnete und zueinander komplementär ausgebildete vierte Oberflächenstrukturierungen aufweisen, die dazu ausgebildet sind, in unterschiedlichen Winkelstellungen formschlüssig ineinander einzugreifen, wobei das erste weitere Federmittel vorzugsweise einstückig mit einer der Drehgelenkkomponenten ausgebildet ist, wobei das erste weitere Federmittel besonders bevorzugt in axialer Richtung aus der Drehgelenkkomponente hervorsteht. Hierdurch wird auf vorteilhafte Weise sichergestellt, dass sich die Klemmen in vorgespannten Zustand bezüglich der Drehachse gegeneinander verdrehen lassen und in verspanntem Zustand bezüglich der Drehachse relativ zueinander fixiert sind. Das erste weitere Federmittel beabstandet dabei die Drehgelenkkomponenten des Drehgelenks. Vorzugsweise ist vorgesehen, dass die erste Klemme eine der Drehgelenkkomponenten aufweist oder dass die zweite Klemme eine der Drehgelenkkomponenten aufweist.

Das Zugelement ist vorzugsweise zentral durch die das Drehgelenk bildenden Drehgelenkkomponenten geführt. Hierzu weisen die Drehgelenkkomponenten ebenfalls die Bohrung auf. Das erste weitere Federmittel ist vorzugsweise in der Art einer Wellenfeder ausgeführt. Vorzugsweise ist vorgesehen, dass das erste weitere Federmittel konzentrisch zueinander und um die axiale Achse angeordnete Ringe oder Ringelemente aufweist, die über in axialer Richtung verlaufende Stege miteinander verbunden sind. Denkbar ist, dass das erste weitere Federmittel als biegebelastbare oder biegebelastete und daher biegeelastische Zylinderbalkenfeder ausgeführt ist. Das erste weitere Federmittel ist vorzugsweise einstückig mit einer Drehgelenkkomponente verbunden. Hierzu ist denkbar, dass das erste weitere Federmittel in axialer Richtung aus der Drehgelenkkomponente herausragt. Vorzugsweise ist hierzu vorgesehen, dass das erste weitere Federmittel ein Ringelement aufweist, welches über Stege mit der Drehgelenkkomponente einstückig verbunden ist. Denkbar ist aber auch, dass das erste weitere Federmittel als separates Bauteil ausgeführt ist. Die Drehgelenkkomponenten sind vorzugsweise scheibenförmig ausgeführt und einander in axialer Richtung gegenüberliegend angeordnet. Ferner ist denkbar, dass die vierten Oberflächenstrukturierungen eine gezahnte oder geriffelte Struktur aufweisen. Denkbar ist auch, dass die vierten Oberflächenstrukturierungen als umlaufende Zahnkränze ausgeführt sind.

Gemäß einer weiteren bevorzugten Ausführungsform der vorliegenden Erfindung ist vorgesehen, dass das Schwenkgelenk zumindest zwei zueinander um die Schwenkachse schwenkbar gelagerte und aneinander angrenzende Schwenkgelenkkomponenten umfasst, die mittels des Zugelementes relativ zueinander in unterschiedlichen Winkelstellungen arretierbar sind, wobei zwischen den Schwenkgelenkkomponenten ein zweites weiteres Federmittel angeordnet ist, welches dazu ausgebildet ist, die Schwenkgelenkkomponenten in axialer Richtung voneinander weg zu spannen, wobei die Schwenkgelenkkomponenten einander gegenüberliegend angeordnete und zueinander komplementär ausgebildete fünfte Oberflächenstrukturierungen aufweisen, die dazu ausgebildet sind, in unterschiedlichen Winkelstellungen formschlüssig ineinander einzugreifen.

Hierdurch wird auf vorteilhafte Weise bewirkt, dass sich die Klemmen in vorgespanntem Zustand bezüglich der Schwenkachse gegeneinander verschwenken lassen und in verspanntem Zustand bezüglich der Schwenkachse relativ zueinander fixiert sind. Das zweite weitere Federmittel beabstandet dabei die Schwenkgelenckomponenten des Schwenkgelenks. Vorzugsweise ist vorgesehen, dass die erste Klemme eine der Schwenkgelenkkomponenten aufweist. Hierzu ist insbesondere vorgesehen, dass die zweite Klemme eine Drehgelenkkomponente aufweist. Alternativ ist vorzugsweise vorgesehen, dass die zweite Klemme eine der Schwenkgelenkkomponenten aufweist. Hierzu ist insbesondere vorgesehen, dass die erste Klemme eine Drehgelenkkomponente aufweist.

Das Zugelement ist vorzugsweise zentral durch die das Schwenkgelenk bildenden Schwenkgelenkkomponenten geführt. Hierzu weisen die Schwenkgelenkkomponenten ebenfalls die Bohrung auf. Das zweite weitere Federmittel ist vorzugsweise als Blattfeder ausgeführt. Insbesondere ist vorgesehen, dass die Blattfeder eine mittig angeordnete weitere Öffnung aufweist, durch welche das Zugelement durchgeführt ist.

Die Schwenkgelenkkomponenten sind vorzugsweise scheibenförmig ausgeführt und dabei so angeordnet, dass eine Schwenkgelenkkomponente mit einer im Wesentlichen zylindrisch-konvexen Seite einer im Wesentlichen zylindrisch-konkaven Seite der anderen Schwenkgelenkkomponente in axialer Richtung gegenüberliegt. Ferner ist denkbar, dass die fünften Oberflächenstrukturierungen eine gezahnte oder geriffelte Struktur aufweisen.

Denkbar ist, dass eine Schwenkgelenkkomponente einstückig mit einer Klemmbacke einer Klemme verbunden ist und dass die andere Schwenkgelenkkomponente einstückig mit einer Drehgelenkkomponente verbunden ist. Hierzu ist vorzugsweise vorgesehen, dass die andere Drehgelenkkomponente, also die Drehgelenckomponente, welche nicht einstückig mit der Schwenkgelenkkomponente verbunden ist, einstückig mit einer Klemmbacke der anderen Klemme, also der Klemme, welche keine Drehgelenkkomponente aufweist, verbunden ist. Insbesondere ist vorgesehen, dass zwischen der ersten Klemme und der zweiten Klemme lediglich das zweite weitere Federmittel sowie ein einstückiges Bauelement, welches eine Schwenkgelenkkomponente und eine Drehgelenkkomponente aufweist, angeordnet ist.

Gemäß einer weiteren bevorzugten Ausführungsform der vorliegenden Erfindung ist vorgesehen, dass das Zugelement endseitig in einem Kugelgelenk der zweiten Klemme, insbesondere der vierten Klemmbacke beweglich geführt ist, wobei das Zugelement ein, zumindest bereichsweise kugelförmig ausgebildetes, Kopfende aufweist, wobei das Kopfende in einer Gelenkpfanne des Schwenkgelenks beweglich geführt ist. Dies ermöglicht auf besonders vorteilhafte Weise eine hohe Beweglichkeit der Klemmvorrichtung. Das Kugelgelenk ermöglicht es, dass das Zugelement eine Drehung um die Drehachse und einer Schwenkbewegung um die Schwenkachse folgen kann. Insbesondere ist so auf vorteilhafte Weise bewirkt, dass das Zugelement in einer 360°-Bewegung bewegt werden kann. Das Zugelement ist nicht auf eine Schwenkbewegung in einer einzigen Ebene reduziert. Weiterhin ist so auf vorteilhafte Weise erreicht, dass nur sehr wenige Bauteile zur Realisierung des Schwenkgelenks verwendet werden müssen. Es ist auf vorteilhafte Weise nicht notwendig, dass eine Achswelle in einem Achssitz angeordnet werden muss. Geführt und gelagert werden im Sinne der vorliegenden Erfindung synonym gebraucht.

Um sicherzustellen, dass eine Fixierung und Verspannung der Komponenten der Klemmvorrichtung einfach und mit wenig Bewegungsaufwand durchführbar ist, ist vorzugsweise vorgesehen, dass das Kopfende des Zugelementes zumindest eine Fixiernut aufweist, in die ein der Gelenkpfanne hervorstehender Vorsprung derart eingreift, dass eine Rotation des Zugelementes um die axiale Richtung zumindest eingeschränkt ist. Durch die Einschränkung der Rotation des Zugelementes um die axiale Richtung ist sichergestellt, dass das Anziehmittel mit dem Handrad auf das Zugelement gedreht werden kann.

Weitere Einzelheiten, Merkmale und Vorteile der Erfindung ergeben sich aus den Zeichnungen sowie aus der nachfolgenden Beschreibung von bevorzugten Ausführungsformen anhand der Zeichnungen. Die Zeichnungen illustrieren dabei lediglich beispielhafte Ausführungsform der Erfindung, welche den Erfindungsgedanken nicht einschränken.

Es zeigen:
- Fig. 1:: eine schematische Explosionsdarstellung einer Klemmvorrichtung gemäß einer beispielhaften Ausführungsform der vorliegenden Erfindung,
- Fig. 2 - 7:: schematische Darstellungen einer Klemmvorrichtung gemäß einer beispielhaften Ausführungsform der vorliegenden Erfindung in unterschiedlichen Dreh- bzw. Schwenkpositionen,
- Fig. 8a, b: eine schematische schnittbildliche Darstellung bzw. eine schematische Explosionszeichnung eines Details einer Klemmvorrichtung gemäß einer beispielhaften Ausführungsform der vorliegenden Erfindung,
- Fig. 9 a - c: schematische Darstellungen eines Teils einer Klemmvorrichtung gemäß einer beispielhaften Ausführungsform der vorliegenden Erfindung,
- Fig. 10 a, b: schematische Darstellungen eines Anziehmittels einer Klemmvorrichtung gemäß einer beispielhaften Ausführungsform der vorliegenden Erfindung,
- Fig. 11: eine schematische Darstellung der dritten Klemmbacke einer Klemmvorrichtung gemäß einer beispielhaften Ausführungsform der vorliegenden Erfindung,
- Fig. 12: einen externen Fixateur, welcher zwei Klemmvorrichtungen gemäß einer beispielhaften Ausführungsform der vorliegenden Erfindung aufweist,
- Fig. 13 a, b: ein Stabelement in schnittbildlicher Darstellung bzw. in perspektivischer Darstellung zur Verwendung mit einer Klemmvorrichtung gemäß einer beispielhaften Ausführungsform der vorliegenden Erfindung.

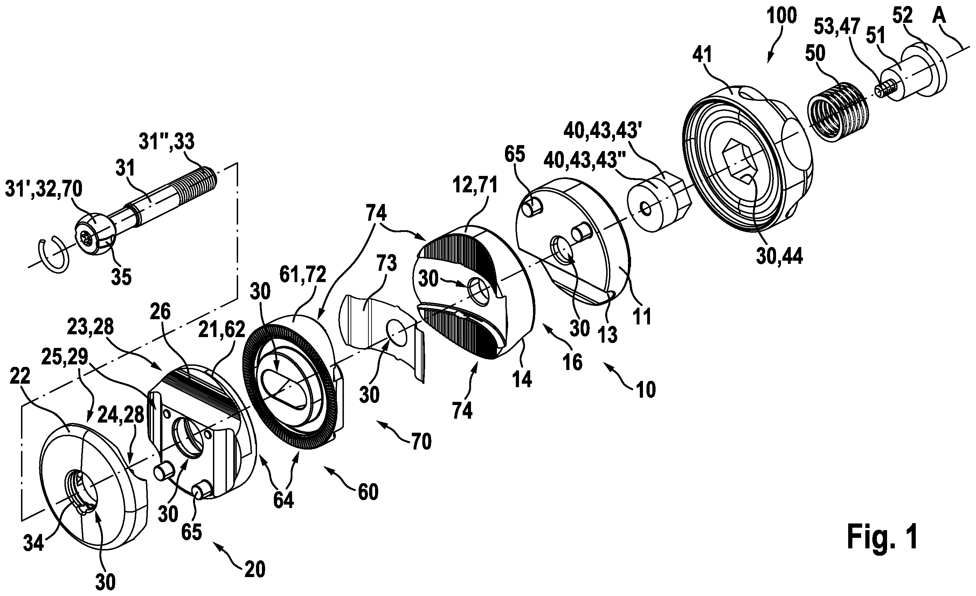

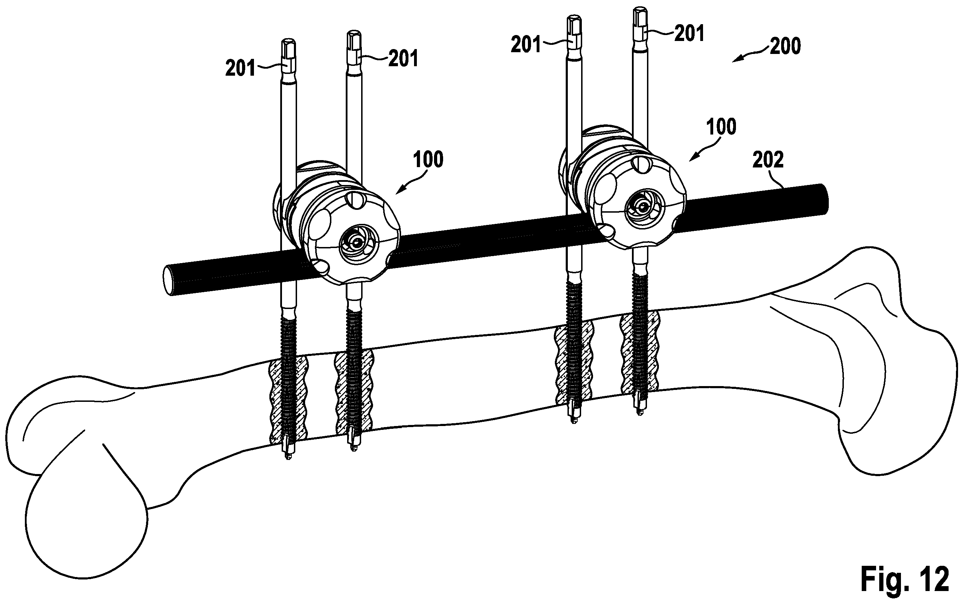

Figur 1 zeigt eine Klemmvorrichtung 100 gemäß einer bevorzugten Ausführungsform der vorliegenden Erfindung in einer schematischen Explosionsdarstellung. Die Klemmvorrichtung 100 ist dazu vorgesehen, Pins 201 und Stabelemente 202 eines externen Fixateurs 200 (siehe Figur 12) zu verbinden. Ein externer Fixateur 200 wird beispielsweise zur vorläufigen Versorgung von Knochenbrüchen eingesetzt. Hierzu werden Pins 201 in Knochenfragmenten des gebrochenen Knochens verankert, beispielsweise eingeschraubt. Mindestens ein Stabelement 202 verbindet die Pins 201 miteinander und fixiert somit die Knochenfragmente relativ zueinander. Die Pins 201 und das mindestens ein Stabelement 202 werden hierzu mit Klemmvorrichtungen 100 verbunden.

Eine Klemmvorrichtung 100 weist eine erste Klemme 10 sowie eine zweite Klemme 20 auf. Die erste Klemme 10 wird durch eine erste Klemmbacke 11 und eine zweite Klemmbacke 12 gebildet. Die erste Klemmbacke 11 und die zweite Klemmbacke 12 sind einstückig vorzugsweise aus einem Metall, besonders bevorzugt aus Aluminium, Stahl oder Titan hergestellt. Sowohl die erste Klemmbacke 11 als auch die zweite Klemmbacke 12 weisen jeweils eine Nut 13, 14 auf, welche entlang der axialen Richtung A gegenüberliegend so angeordnet sind, dass die Nuten 13, 14 eine erste offene Aufnahmenut 16 zur Aufnahme eines Stabelementes 202 bilden. Die zweite Klemme 20 wird durch eine dritte Klemmbacke 21 und eine vierte Klemmbacke 22 gebildet. Die dritte Klemmbacke 21 und die vierte Klemmbacke 22 sind ebenfalls einstückig vorzugsweise aus einem Metall, insbesondere aus Aluminium, Stahl oder Titan hergestellt. Die dritte Klemmbacke 21 und die vierte Klemmbacke 22 weisen Nuten 23, 24 auf, welche entlang der axialen Richtung A gegenüberliegend so angeordnet sind, dass sie eine zweite offene Aufnahmenut 28 zur Aufnahme eines Stabelementes 202 bilden. Die dritte Klemmbacke 21 und vierte Klemmbacke 22 weisen ferner weitere Nuten 25 auf. Die weiteren Nuten 25, hier zwei weitere Nuten 25, sind in einem 90° Winkel zur zweiten offenen Aufnahmenut 28 angeordnet und liegen entlang der axialen Richtung A einander gegenüber. Die weitere Nuten 25 bilden weitere Aufnahmenuten 29, welche dazu vorgesehen sind, dass Pins 201 in ihnen angeordnet werden.

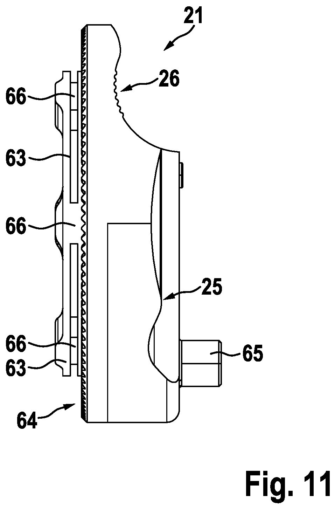

Um die Klemmbacken 11, 12, 21, 22 gegen ein Verdrehen zu sichern und um sicherzustellen, dass die Nuten 13, 14, 23, 24 und die weiteren Nuten 25 so gegenüberliegend angeordnet sind, dass Aufnahmenuten 16, 28 bzw. weitere Aufnahmenuten 29 gebildet werden, sind ineinandergreifende Strukturen 65 an den sich gegenüberliegenden Oberflächen der Klemmbacken 11, 12 der ersten Klemme 10 und den sich gegenüberliegenden Oberflächen der Klemmbacken 21, 22 der zweiten Klemme 20 vorgesehen. Im vorliegenden Fall weisen die ineinandergreifenden Strukturen 65 Vorsprünge auf, welche in Vertiefungen eingreifen (siehe hierzu insbesondere Figur 11).

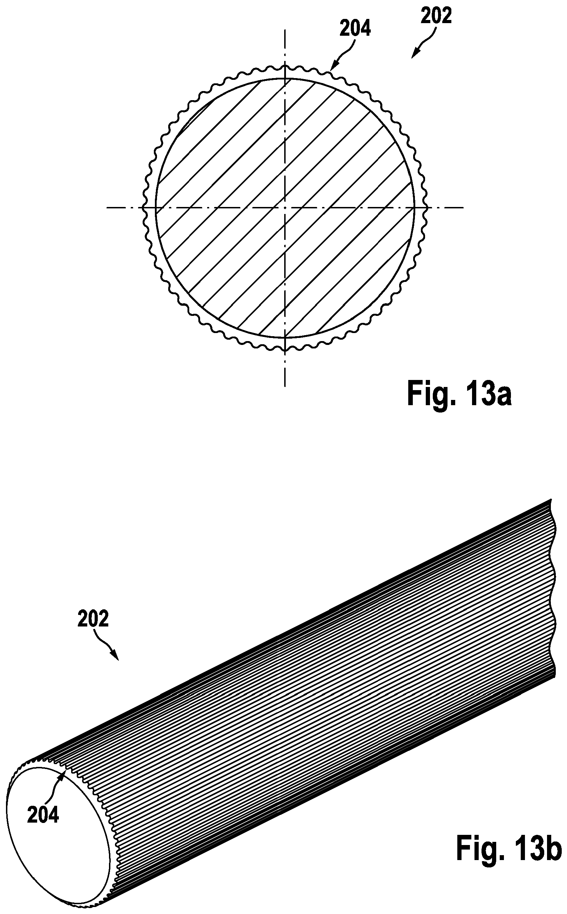

Sind in den Aufnahmenuten 16, 28 Stabelemente 202 angeordnet, so greift eine erste Oberflächenstrukturierung 15 der ersten Klemme 10 bzw. eine zweite Oberflächenstrukturierung 26 der zweiten Klemme 20 in eine erste weitere Oberflächenstrukturierung 204 der Stabelemente 202 (siehe insbesondere Figur 13, welche einen Querschnitt eines Stabelementes 202 zeigt) ein und sorgt für eine formschlüssige Fixierung der Stabelemente 202 in den Aufnahmenuten 16, 28. Vorzugsweise ist vorgesehen, dass die weiteren Aufnahmenuten 29 dritte Oberflächenstrukturierungen aufweisen, welche in zweite weitere Oberflächenstrukturierungen der Pins 201 eingreifen und die Pins 201 formschlüssig in den weiteren Aufnahmenuten 29 fixieren.

Um einen festen Sitz der Stabelemente 202 bzw. Pins 201 in der Klemmvorrichtung 100 zu gewährleisten, können die Klemmbacken 11, 12 der ersten Klemme 10 und die Klemmbacken 21, 22 gegeneinander verspannt werden.

Die erste Klemme 10 und die zweite Klemme 20 weisen eine durchgehenden Bohrung 30 auf, in welcher ein Zugelement 31 entlang einer axialen Richtung A angeordnet ist. Das Zugelement 31 weist einen länglichen, bolzenförmigen Grundkörper auf, an dessen erstem Ende 31' ein zumindest teilweise kugelförmiges Kopfende 32 angeordnet ist. An einem zweiten Ende 31", welches dem ersten Ende 31' gegenüberliegt, ist ein Gewinde 33 angeordnet. Das Zugelement 31 ist mit seinem kugelförmigen Kopfende 32 in einer Gelenkpfanne 34 der zweiten Klemme 20 angeordnet. Um eine Rotation des Zugelementes 31 relativ zur Gelenkpfanne 34 um die axiale Richtung A zu verhindern, greift ein hervorstehender Vorsprung (aus perspektivischen Gründen hier nicht sichtbar) der Gelenkpfanne 34 in eine Fixiernut 35 des kugelförmigen Kopfendes 32 ein.

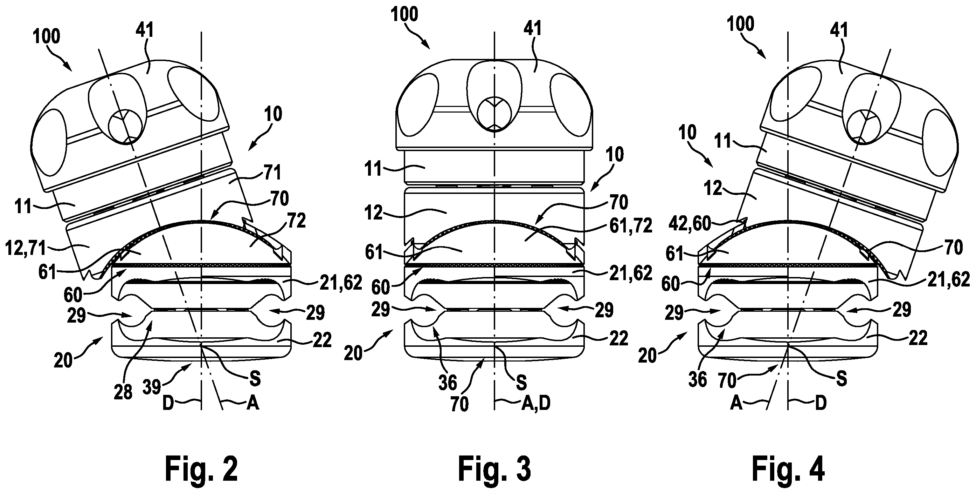

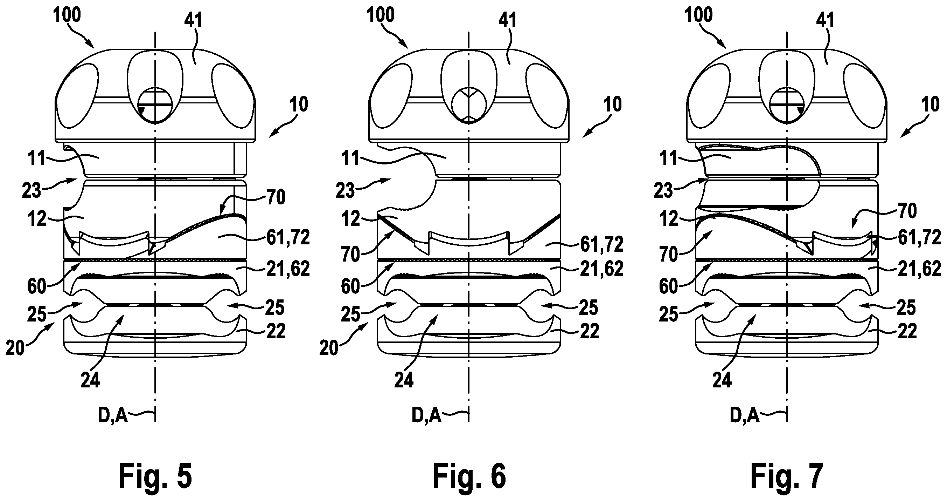

Um Pins 201 und Stabelemente 202 so aufnehmen zu können, dass die Knochenfragmente in einer anatomisch korrekten Position zueinander fixiert sind, muss die Klemmvorrichtung 100 dazu vorgesehen sein, Pins 201 und Stabelemente 202 mit unterschiedlichen und situationsabhängigen Raumausrichtung aufzunehmen. Zur Lageanpassung der ersten Aufnahmenut 16 relativ zur zweiten Aufnahmenut 28 bzw. zu den weiteren Aufnahmenuten 29 (siehe Figuren 2 bis 7) weist die Klemmvorrichtung 100 ein Drehgelenk 60 auf, welches es erlaubt, die erste Klemme 10 um eine Drehachse D relativ zur zweiten Klemme 20 zu drehen.

Das Drehgelenk 60 weist hierfür drehbar gelagerte und aneinander angrenzende Drehgelenkkomponenten 61, 62 auf. Eine der Drehgelenkkomponenten 62 wird durch die dritte Klemmbacke 21 gebildet. Der dritten Klemmbacke 21 liegt in axialer Richtung A die andere Drehgelenkkomponente 61 gegenüber. Beide Drehgelenkkomponenten 61, 62 weisen jeweils eine vierte Oberflächenstrukturierung 64 auf. Die vierten Oberflächenstrukturierungen 64 sind hier in Form eines Zahnkranzes ausgeführt und in der Lage, in unterschiedlichen Winkelstellungen das Drehgelenk 60 formschlüssig ineinanderzugreifen. Um eine Drehbewegung des Drehgelenk 60 zu ermöglichen, sorgt ein erstes weiteres Federmittel 63 für eine Beabstandung der vierten Oberflächenstrukturierungen 64. Figur 11 zeigt das erste weitere Federmittel 63 im Detail. Das erste weitere Federmittel 63 ist einstückig mit der dritten Klemmbacke 21 verbunden. Es weist in axialer Richtung A aus der dritten Klemmbacke 21 hervorspringende Stege 66 auf, welche einen umlaufenden Ring 67 tragen. Der umlaufende Ring 67 beabstandet die Drehgelenkkomponenten 61, 62 voneinander, sodass die vierten Oberflächenstrukturierungen 64 nicht ineinandergreifen. Werden die Drehgelenkkomponenten 61, 62 in axialer Richtung A aufeinander zugeschoben, so biegt sich das erste weitere Federmittel 63 und lässt einen Formschluss der vierten Oberflächenstrukturierungen 64 zu. Damit ist die Klemmvorrichtung 100 gegen weitere Drehbewegungen um die Drehachse D gesichert.

In der gezeigten Ausführungsform der Klemmvorrichtung ist eine Drehgelenkkomponente 62 durch die dritte Klemmbacke 21 realisiert.

Zur weiteren Lageanpassung der ersten Aufnahmenut 16 relativ zur zweiten Aufnahmenut 28 bzw. den weiteren Aufnahmenuten 29 weist die Klemmvorrichtung 100 ferner ein Schwenkgelenk 70 zum Verschwenken der ersten Klemme 10 relativ zur zweiten Klemme 20 um eine Schwenkachse S auf, welche senkrecht auf der Drehachse D angeordnet ist. Das Schwenkgelenk 70 wird durch Schwenkgelenckomponenten 71, 72 und ein zweites weiteres Federmittel 73 gebildet. Eine Schwenkgelenkkomponente 71 weist eine konkav-zylindrische Oberfläche auf, welche einer konvex-zylindrischen Oberfläche der anderen Schwenkgelenkkomponente 72 gegenüberliegend angeordnet ist. Die Mittelpunkte der konkav- bzw. konvex-zylindrischen Form der Oberflächen liegen im Wesentlichen im Zentrum des kugelförmigen Kopfendes 32 des Zugelementes 31. Auf den konkav-zylindrischen bzw. konvex-zylindrischen Oberflächen der Schwenkgelenkkomponenten 71, 72 ist jeweils eine fünfte Oberflächenstrukturierung 74 angeordnet, welche dazu ausgebildet sind, in unterschiedlichen Winkelstellungen des Schwenkgelenks 70 ineinanderzugreifen. Um eine Schwenkbewegung des Schwenkgelenks 70 zu ermöglichen, sorgt das zweite weitere Federmittel 73 für eine Beabstandung der fünften Oberflächenstrukturierungen 74. Das zweite weitere Federmittel 73 ist hier als Blattfeder vorgesehen, welche eine weitere Öffnung 75 in Form eines Langlochs aufweist, welches das Zugelement 31 durchgreift. Werden die Schwenkgelenckomponenten 71, 72 entgegen der Federkraft des zweiten weiteren Federmittels 73 aufeinander zugedrückt, so greifen die fünften Oberflächenstrukturierungen 74 formschlüssig ineinander und fixieren die erste Klemme 10 und die zweite Klemme 20 relativ zueinander bezüglich der Schwenkachse S.

In der gezeigten Ausführungsform der Klemmvorrichtung 100 ist eine Schwenkgelenkkomponente 71 durch die zweite Klemmbacke 12 realisiert. Ferner ist die andere Schwenkgelenkkomponente 72 mit einer Drehgelenkkomponente 61, welche nicht Teil der dritten Klemmbacke 21 ist, einstückig verbunden.

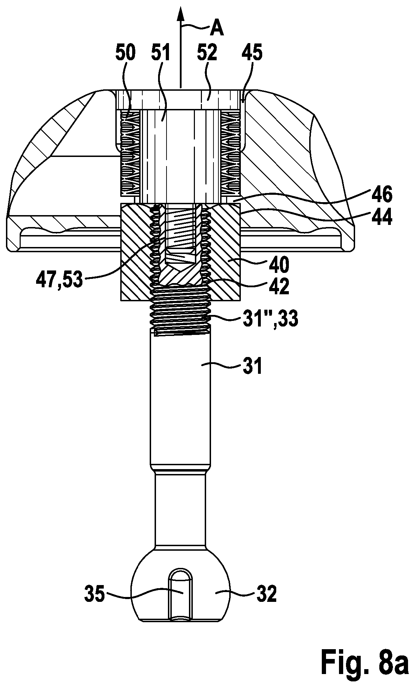

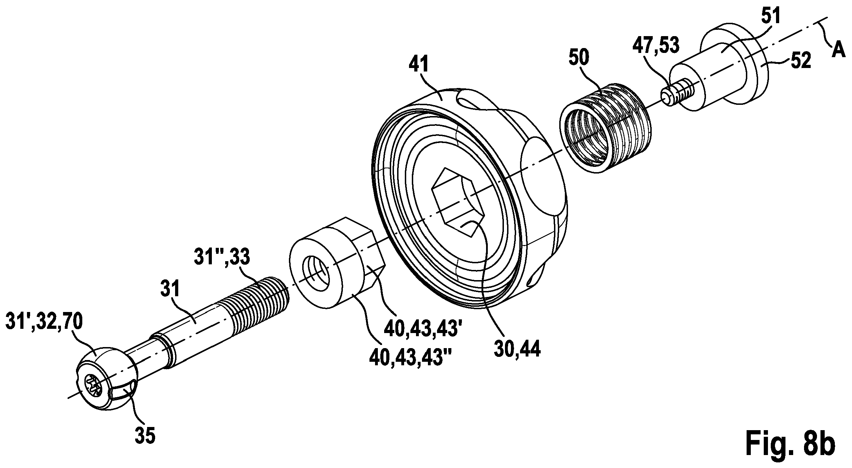

Zur Erreichung einer Fixierung der ersten Klemme 10 relativ zur zweiten Klemme 20 bezüglich der Drehachse D und der Schwenkachse S sowie zur Erreichung einer Verspannung der ersten Klemme 10 und der zweiten Klemme 20 greift das Gewinde 33 des Zugelementes 31 in ein Gegengewinde eines Anziehmittels 40 ein (siehe insbesondere Figur 8). Das Anziehmittel 40 weist einen Eingriffteil 43 auf, welcher formschlüssig in Eingriff mit einer Aufnahme 44 eines Handrades 41 der Klemmvorrichtung 100 steht. Wird das Handrad 41 gedreht, so dreht sich das Anziehmittel 40 mit und das Gewinde 33 des Zugelementes 31 wird in das Gegengewinde des Anziehmittels 40 hinein- oder aus diesem herausgedreht. Die erste Klemmbacke 11, die zweite Klemmbacke 12 mit einer der Schwenkgelenkkomponenten 71, die andere Schwenkgelenkkomponente 72 mit einer der Drehgelenckomponenten 61 und die dritte Klemmbacke 21 mit der anderen Drehgelenkkomponente 62 sind dabei zwischen der vierten Klemmbacke 22 und dem Handrad 41 angeordnet. Wird das Gewinde 33 des Zugelementes 31 in das Gegengewinde des Anziehmittels 40 hineingedreht, so zieht das Zugelement 31 die Klemmbacken 11, 12,21, 22, das Drehgelenk 60 und das Schwenkgelenk 70 zusammen, sodass die Oberflächenstrukturierungen 15, 26, 64, 74 und 204 formschlüssig ineinandergreifen. Damit sind mit einer einzigen Drehbewegung am Handrad 41 das Drehgelenk 60 und das Schwenkgelenk 70 fixiert und die Klemmen 10, 20 verspannt.

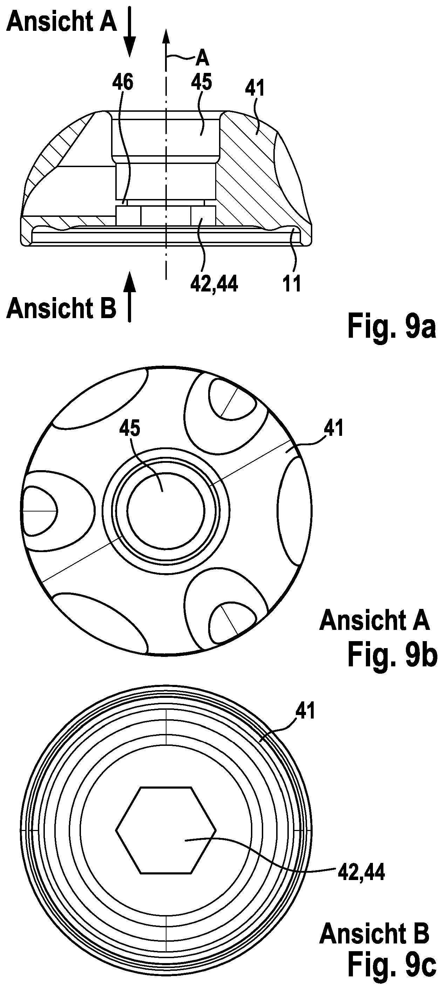

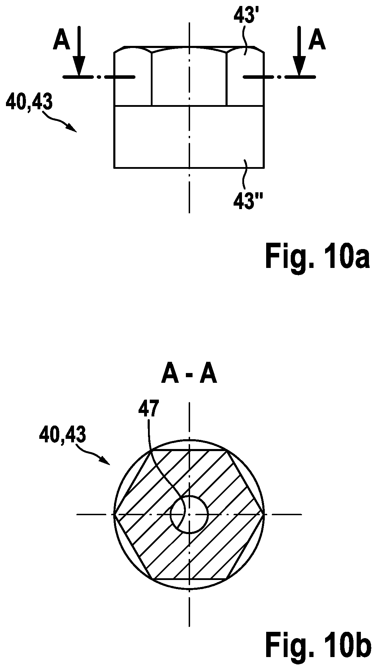

Um einen formschlüssigen Eingriff des Eingriffteils 43 mit dem Handrad 41 zu gewährleisten, ist vorgesehen, dass ein obere Abschnitt 43' des Eingriffteils 43 in einer Aufnahme 44 des Handrades 41 angeordnet ist. Die Aufnahme 44 des Handrades 41 ist dabei fluchtend mit der Bohrung 30 angeordnet. Die Aufnahme 44 des Handrades 41 weist eine Profilierung auf. Diese Profilierung kann wellenförmig, beispielsweise ähnlich einem Torx-Mitnahmeprofil (Torx ist eine registrierte Marke) oder einem Stardrive-Mitnahmeprofil (Stardrive ist eine registrierte Marke) sein. Denkbar sind hier beispielsweise Innensechsrund-, Innenachtrund- oder Innenzehnrund-Profile. Denkbar sind aber auch alle weiteren polygonalen Formen, beispielsweise ein viereckiger, sechseckiger oder achteckiger Querschnitt. Zu erkennen ist dies insbesondere in der Figur 9 c, welche eine Ansicht des Handrades 41 von unten zeigt. Der obere Abschnitt 43' des Eingriffteils 43 ist korrespondierend zur Profilierung der Aufnahme 44 geformt. Figur 10 a zeigt ein Anziehmittel 40. Deutlich zu erkennen ist der profilierte obere Abschnitt 43'. Figur 10 b zeigt einen Schnitt durch den oberen Abschnitt 43', welcher hier einen achteckigen Querschnitt aufweist.

Das Anziehmittel 40 ragt mit einem unteren Abschnitt 43" in die Bohrung 30 hinein. Die Stützstelle 46 ist vorzugsweise einstückig mit dem Handrad 41 verbunden. Denkbar ist aber auch, dass die Stützstelle 46 durch eine kreisringförmige Scheibe, welche in eine Ringnut des Handrades 41 eingelassen ist, gebildet ist.

Die Klemmvorrichtung 100 weist ein Federmittel 50 auf, welches dazu vorgesehen ist, die Klemmbacken 11, 12, 21, 22 der ersten Klemme 10 und der zweiten Klemme 20 vorzuspannen. Hierzu ist das Federmittel 50 in einer Ausnehmung 45 des Handrades 41 angeordnet und liegt an der Stützstelle 46 und an einem Vorspannmittel 51 so an, dass die Federkraft des Federmittels 50 das Vorspannmittel 51 entlang der axialen Richtung A von der Stützstelle 46 wegdrückt. Die Ausnehmung 45 ist dabei fluchtend mit der Aufnahme 44 angeordnet und weist einen runden Querschnitt auf (siehe Figur 9 b, welche das Handrad 41 von oben zeigt). Das Vorspannmittel 51 ist als Sicherungsschraube vorgesehen, welche mit einem weiteren Gewinde 53 in Eingriff mit einem weiteren Gegengewinde 47 des Zugelementes 31 steht. Das Anziehmittel 40 ist hierbei zwischen dem Zugelement 31 und dem Federmittel 50 angeordnet, sodass die Stützstelle 46 zwischen dem Anziehmittel 40 und dem Federmittel 50 angeordnet ist. Das Federmittel 50 stützt sich an einem Schraubenkopf 52 des Vorspannmittel 50 ab. Wird das Vorspannmittel 51 durch das Federmittel 50 entlang der axialen Richtung A bewegt, so wird das Zugelement 31 ebenfalls entlang der axialen Richtung A gezogen was zu einer Vorspannung der Klemmbacken 11, 12, 21 und 22 führt.

### Bezugszeichenliste:

- 10: erste Klemme
- 11: erste Klemmbacke
- 12: zweite Klemmbacke
- 13: Nut
- 14: Nut
- 15: erste Oberflächenstrukturierung
- 16: erste Aufnahmenut
- 20: zweite Klemme
- 21: dritte Klemmbacke
- 22: vierte Klemmbacke
- 23: Nut
- 24: Nut
- 25: weitere Nut
- 26: zweite Oberflächenstrukturierung
- 28: zweite Aufnahmenut
- 29: weitere Aufnahmenut
- 30: durchgehenden Bohrung
- 31: Zugelement
- 31': erstes Ende
- 31": zweites Ende
- 32: Kopfende
- 33: Gewinde
- 34: Gelenkpfanne
- 35: Fixiernut
- 40: Anziehmittel
- 41: Handrad
- 42: erste weitere Bohrung
- 43: Eingriffteil
- 43': obere Abschnitt
- 43": untere Abschnitt in
- 44: Aufnahme
- 45: Ausnehmung
- 46: Stützstelle
- 50: Federmittel
- 51: Vorspannmittel
- 52: Schraubenkopf
- 60: Drehgelenk
- 61: Drehgelenkkomponente
- 62: Drehgelenkkomponente
- 63: erstes weiteres Federmittel
- 64: vierte Oberflächenstrukturierung
- 70: Schwenkgelenk
- 71: Schwenkgelenkkomponente
- 72: Schwenkgelenkkomponente
- 73: zweites weiteres Federmittel
- 74: fünfte Oberflächenstrukturierung
- 75: weitere Öffnung

- 100: Klemmvorrichtung
- 200: externer Fixateur
- 201: Pin
- 202: Stabelement
- 204: erste weitere Oberflächenstrukturierung
- A: axiale Richtung
- D: Drehachse
- S: Schwenkachse

## Patentansprüche

1. Klemmvorrichtung (100) zum Verbinden von Pins (201) und/oder Stabelementen (202) eines externen Fixateurs (200) zur Behandlung von Knochenbrüchen,
aufweisend eine erste Klemme (10) und eine zweite Klemme (20), wobei entlang einer axialen Richtung (A) in einer durchgehenden Bohrung (30) der ersten Klemme (10) und der zweiten Klemme (20) ein, die erste Klemme (10) mit der zweiten Klemme (20) verbindendes, Zugelement (31) vorgesehen ist, wobei das Zugelement (31) mit einem ersten Ende (31') in der zweiten Klemme (20) gelagert ist und an einem zweiten Ende (31"), welches dem ersten Ende (31') gegenüberliegt, ein Gewinde (33) aufweist, das in ein Gegengewinde eines Anziehmittels (40) eingreift, wobei das Zugelement (31) zum Verspannen der ersten Klemme (10) und der zweiten Klemme (20) durch Einschrauben des Gewindes (33) in das Gegengewinde vorgesehen ist, wobei die Klemmvorrichtung (100) ein Federmittel (50) zum Vorspannen der ersten Klemme (10) und der zweiten Klemme (20) aufweist, **dadurch gekennzeichnet, dass** das Anziehmittel (40) zwischen dem Federmittel (50) und dem Zugelement (31) angeordnet ist, wobei die Klemmvorrichtung (100) ein Handrad (41) aufweist, wobei das Handrad (41) zum Drehen des Anziehmittels (40) mit dem Anziehmittel (40) in Eingriff steht, wobei das Anziehmittel (40) entlang der axialen Richtung (A) an dem Handrad (41) anliegend angeordnet ist, wobei das Anziehmittel (40) zwischen dem Handrad (41) und der ersten Klemme (10) angeordnet ist, wobei das Gegengewinde des Anziehmittels (40) in einer mit der durchgehenden Bohrung (30) fluchtenden ersten weiteren Bohrung (42) des Anziehmittels (40) angeordnet ist, wobei ein Eingriffteil (43) des Anziehmittels (40) formschlüssig in Eingriff mit einer Aufnahme (44) des Handrades (41) steht, wobei das Handrad (41) eine Ausnehmung (45) aufweist, wobei das Federmittel (50) innerhalb der Ausnehmung (45) angeordnet ist, wobei das Federmittel (50) an einer Stützstelle (46) des Handrades (41) und an einem Vorspannmittel (51) anliegend angeordnet ist, wobei das Vorspannmittel (51) in Eingriff mit dem Zugelement (31) steht, wobei das Federmittel (50) so angeordnet ist, dass eine Federkraft des Federmittels (50) das Zugelement (31) entlang der axialen Richtung (A) in Richtung des Handrades (41) zieht.

2. Klemmvorrichtung (100) nach Anspruch 1, **dadurch gekennzeichnet, dass** die erste Klemme (10) eine erste Klemmbacke (11) und eine zweite Klemmbacke (12) aufweist und dass die zweite Klemme (20) eine dritte Klemmbacke (21) und eine vierte Klemmbacke (22) aufweist, wobei die erste Klemmbacke (11), die zweite Klemmbacke (12), die dritte Klemmbacke (21) sowie die vierte Klemmbacke (22) jeweils eine Nut (13, 14, 23, 24) aufweisen, wobei durch die Nuten (13, 14) der ersten Klemmbacke (11) und der zweiten Klemmbacke (12) eine offene erste Aufnahmenut (16) zum Aufnehmen eines Stabelementes (202) gebildet ist und durch die Nuten (22, 24) der dritten Klemmbacke (21) und der vierten Klemmbacke (22) eine offene zweite Aufnahmenut (28) zur Aufnahme eines Stabelementes (202) eines gebildet ist, wobei die dritte Klemmbacke (21) und die vierte Klemmbacke (22) jeweils eine, vorzugsweise zwei weiteren Nuten (25) aufweisen, wobei durch die weiteren Nuten (25) der dritten Klemmbacke (21) und der vierten Klemmbacke (22) mindestens eine, vorzugsweise zwei, offene weitere Aufnahmenuten (29) zur Aufnahme eines Pins (201) gebildet sind,
wobei die erste Aufnahmenut (16) zumindest abschnittsweise vorzugsweise mit ersten Oberflächenstrukturierungen (15) versehen ist, die dazu ausgebildet sind, in komplementär ausgebildete erste weitere Oberflächenstrukturierungen (204) eines Stabelements (202) formschlüssig einzugreifen und/oder die zweite Aufnahmenut (28) zumindest abschnittsweise vorzugsweise mit zweiten Oberflächenstrukturierungen (26) versehen ist, die dazu ausgebildet sind, in komplementär ausgebildete erste weitere Oberflächenstrukturierungen (204) eines Stabelements (202) formschlüssig einzugreifen und/oder die weitere Aufnahmenut (29) bzw. die weiteren Aufnahmenuten (29) zumindest abschnittsweise vorzugsweise mit dritten Oberflächenstrukturierungen versehen ist, die dazu ausgebildet sind, in komplementär ausgebildete zweite weitere Oberflächenstrukturierungen (203) eines Pins (201) formschlüssig einzugreifen.

3. Klemmvorrichtung (100) nach einem der vorhergehenden Ansprüche, **dadurch gekennzeichnet, dass** die Ausnehmung (45) mit der Bohrung (30) fluchtend angeordnet ist.

4. Klemmvorrichtung (100) nach einem der vorhergehenden Ansprüche, **dadurch gekennzeichnet, dass** das Vorspannmittel (51) eine Sicherungsschraube aufweist, deren weiteres Gewinde (53) in Eingriff mit einem weiteren Gegengewinde (47) des Zugelementes (31) steht, wobei vorzugsweise das Federmittel (50) an einer der Stützstelle (46) zugewandten Unterseite eines Schraubenkopfes (52) der Sicherungsschraube anliegend angeordnet ist.

5. Klemmvorrichtung (100) nach einem der vorhergehenden Ansprüche, **dadurch gekennzeichnet, dass** die Stützstelle (46) zwischen der Aufnahme (44) des Handrades (41) und der Ausnehmung (45) des Handrades (41) angeordnet ist, wobei die Stützstelle (46) vorzugsweise einstückig mit dem Handrad (41) ausgebildet ist oder wobei die Stützstelle (46) eine ringförmige Scheibe aufweist, welche in eine Ringnut des Handrades (41) eingesetzt ist.

6. Klemmvorrichtung (100) nach einem der vorhergehenden Ansprüche, **dadurch gekennzeichnet, dass** die Aufnahme (44) einen profilierten Querschnitt, vorzugsweise einen wellenförmigen und/oder eckigen, besonders bevorzugt quadratischen, sechs- oder achteckigen Querschnitt aufweist, wobei der Eingriffteil (43) an einem oberen Abschnitt (43') des Anziehmittels (40) angeordnet ist, wobei der obere Abschnitt (43') einen profilierten Querschnitt, vorzugsweise einen wellenförmigen und/oder eckigen, besonders bevorzugt quadratischen oder sechseckigen, Querschnitt aufweist.

7. Klemmvorrichtung (100) nach Anspruch 6, **dadurch gekennzeichnet, dass** das Anziehmittel einen unteren Abschnitt (43") aufweist, wobei der untere Abschnitt (43") in der Bohrung (30) angeordnet ist, wobei der untere Abschnitt (43") vorzugsweise einen runden Querschnitt aufweist.

8. Klemmvorrichtung (100) nach einem der vorhergehenden Ansprüche, **dadurch gekennzeichnet, dass** die erste Klemme (10) und die zweite Klemme (20) über ein Drehgelenk (60) um eine Drehachse (D) zueinander drehbar und über ein Schwenkgelenk (70) um eine senkrecht zur Drehachse (D) angeordnete Schwenkachse (S) zueinander schwenkbar gelagert sind, wobei das Zugelement (31) zum Fixieren der ersten Klemme (10) relativ zur zweiten Klemme (20) bezüglich der Drehachse (D) und der Schwenkachse (S) durch Einschrauben des Gewindes (33) in das Gegengewinde vorgesehen ist.

9. Klemmvorrichtung (100) nach Anspruch 8, **dadurch gekennzeichnet, dass** das Drehgelenk (60) zumindest zwei zueinander um die Drehachse (D) drehbar gelagerte und aneinander angrenzende Drehgelenkkomponenten (61, 62) umfasst, die mittels des Zugelements (31) relativ zueinander in unterschiedlichen Winkelstellungen arretierbar sind, wobei zwischen den Drehgelenkkomponenten (61, 62) ein erstes weiteres Federmittel (63) angeordnet ist, welches dazu ausgebildet ist, die Drehgelenkkomponenten (61, 62) in axialer Richtung (A) voneinander weg zu spannen,
wobei die Drehgelenkkomponenten (61, 62) einander gegenüberliegend angeordnete und zueinander komplementär ausgebildete vierte Oberflächenstrukturierungen (64) aufweisen, die dazu ausgebildet sind, in unterschiedlichen Winkelstellungen formschlüssig ineinander einzugreifen,
wobei das erste weitere Federmittel (63) vorzugsweise einstückig mit einer der Drehgelenkkomponenten (61, 62) ausgebildet ist, wobei das erste weitere Federmittel (63) besonders bevorzugt in axialer Richtung (A) aus der Drehgelenkkomponente (61, 62) hervorsteht.

10. Klemmvorrichtung (100) nach einem der Ansprüche 8 bis 9, **dadurch gekennzeichnet, dass** das Schwenkgelenk (70) zumindest zwei zueinander um die Schwenkachse (S) schwenkbar gelagerte und aneinander angrenzende Schwenkgelenkkomponenten (71, 72) umfasst, die mittels des Zugelementes (31) relativ zueinander in unterschiedlichen Winkelstellungen arretierbar sind, wobei zwischen den Schwenkgelenkkomponenten (71, 72) ein zweites weiteres Federmittel (73) angeordnet ist, welches dazu ausgebildet ist, die Schwenkgelenkkomponenten (71, 72) in axialer Richtung (A) voneinander weg zu spannen,
wobei die Schwenkgelenkkomponenten (71, 72) einander gegenüberliegend angeordnete und zueinander komplementär ausgebildete fünfte Oberflächenstrukturierungen (74) aufweisen, die dazu ausgebildet sind, in unterschiedlichen Winkelstellungen formschlüssig ineinander einzugreifen.

11. Klemmvorrichtung (100) nach einem der vorhergehenden Ansprüche, **dadurch gekennzeichnet, dass** das Zugelement (31) endseitig in einem Kugelgelenk der zweiten Klemme (20), insbesondere der vierten Klemmbacke (22) beweglich geführt ist, wobei das Zugelement (31) ein, zumindest bereichsweise kugelförmig ausgebildetes, Kopfende (32) aufweist, wobei das Kopfende (32) in einer Gelenkpfanne (34) des Schwenkgelenk (70) beweglich geführt ist.

12. Klemmvorrichtung (100) nach Anspruch 11, **dadurch gekennzeichnet, dass** das Kopfende (32) des Zugelementes (31) zumindest eine Fixiernut (35) aufweist, in die ein der Gelenkpfanne (34) hervorstehender Vorsprung derart eingreift, dass eine Rotation des Zugelementes (31) um die axiale Richtung (A) zumindest eingeschränkt ist.

## Claims

1. Clamping device (100) for connecting pins (201) and/or rod elements (202) of an external fixator (200) for the treatment of bone fractures, comprising a first clamp (10) and a second clamp (20), wherein a traction element (31) connecting the first clamp (10) to the second clamp (20) is provided along an axial direction (A) in a continuous bore (30) of the first clamp (10) and the second clamp (20), wherein the traction element (31) is mounted with a first end (31') in the second clamp (20) and has, at a second end (31") opposite the first end (31'), a thread (33) which engages into a mating thread of a tightening means (40), wherein the traction element (31) is provided for clamping the first clamp (10) and the second clamp (20) by screwing the thread (33) into the mating thread, wherein the clamping device (100) has a spring means (50) for pre-tensioning the first clamp (10) and the second clamp (20) , **characterized by** the tightening means (40) which is arranged between the spring means (50) and the traction element (31), wherein the clamping device (100) has a handwheel (41), wherein the handwheel (41) is in engagement with the tightening means (40) for rotating the tightening means (40), wherein the tightening means (40) is arranged abutting against the handwheel (41) along the axial direction (A) , wherein the tightening means (40) is arranged between the handwheel (41) and the first clamp (10), wherein the mating thread of the tightening means (40) is arranged in a first further bore (42) of the tightening means (40) aligned with the continuous bore (30), wherein an engagement part (43) of the tightening means (40) is in form-fitting engagement with a receptacle (44) of the handwheel (41) , wherein the handwheel (41) has a recess (45), wherein the spring means (50) is arranged within the recess (45), wherein the spring means (50) is arranged abutting against a support point (46) of the handwheel (41) and against a biasing means (51), wherein the biasing means (51) is in engagement with the traction element (31) , wherein the spring means (50) is arranged such that a spring force of the spring means (50) pulls the traction element (31) along the axial direction (A) in the direction of the handwheel (41).

2. Clamping device (100) according to claim 1, **characterized by** the first clamp (10) which has a first clamping jaw (11) and a second clamping jaw (12) and that the second clamp (20) has a third clamping jaw (21) and a fourth clamping jaw (22) , wherein the first clamping jaw (11), the second clamping jaw (12), the third clamping jaw (21) and the fourth clamping jaw (22) each have a groove (13, 14, 23, 24), wherein an open first receiving groove (16) for receiving a rod element (202) is formed by the grooves (13, 14) of the first clamping jaw (11) and the second clamping jaw (12) and an open second receiving groove (28) for receiving a rod element (202) is formed by the grooves (22, 24) of the third clamping jaw (21) and the fourth clamping jaw (22) , wherein the third clamping jaw (21) and the fourth clamping jaw (22) each have one, preferably two, further grooves (25), wherein at least one, preferably two, open further receiving grooves (29) for receiving a pin (201) are formed by the further grooves (25) of the third clamping jaw (21) and the fourth clamping jaw (22) , wherein the first receiving groove (16) is provided at least in sections preferably with first surface structurings (15) which are designed to engage in a form-fitting manner into complementarily formed first further surface structurings (204) of a rod element (202) and/or the second receiving groove (28) is provided at least in sections preferably with second surface structurings (26) which are designed to engage in a form-fitting manner into complementarily formed first further surface structurings (204) of a rod element (202) and/or the further receiving groove (29) or the further receiving grooves (29) is/are provided at least in sections preferably with third surface structurings which are designed to engage in a form-fitting manner into complementarily formed second further surface structurings (203) of a pin (201).

3. Clamping device (100) according to one of the preceding claims,
**characterized by** the recess (45) which is arranged in alignment with the bore (30).

4. Clamping device (100) according to one of the preceding claims,
**characterized by** the biasing means (51) which has a locking screw, the further thread (53) of which is in engagement with a further mating thread (47) of the traction element (31), wherein preferably the spring means (50) is arranged abutting against an underside of a screw head (52) of the locking screw facing the support point (46).

5. Clamping device (100) according to one of the preceding claims,
**characterized by** the support point (46) which is arranged between the receptacle (44) of the handwheel (41) and the recess (45) of the handwheel (41), wherein the support point (46) is preferably formed integrally with the handwheel (41) or wherein the support point (46) comprises an annular disc which is inserted into an annular groove of the handwheel (41).

6. Clamping device (100) according to one of the preceding claims,
**characterized by** the receptacle (44) which has a profiled cross-section, preferably a wave-shaped and/or angular, particularly preferably square, hexagonal or octagonal cross-section, wherein the engagement part (43) is arranged on an upper section (43') of the tightening means (40), wherein the upper section (43') has a profiled cross-section, preferably a wave-shaped and/or angular, particularly preferably square or hexagonal, cross-section.

7. Clamping device (100) according to claim 6, **characterized by** the tightening means which has a lower section (43"), wherein the lower section (43") is arranged in the bore (30), wherein the lower section (43") preferably has a round cross-section.

8. Clamping device (100) according to one of the preceding claims,
**characterized by** the first clamp (10) and the second clamp (20) which are mounted rotatably relative to one another via a rotary joint (60) about an axis of rotation (D) and pivotably relative to one another via a swivel joint (70) about a pivot axis (S) arranged perpendicular to the axis of rotation (D), wherein the traction element (31) is provided for fixing the first clamp (10) relative to the second clamp (20) with respect to the axis of rotation (D) and the pivot axis (S) by screwing the thread (33) into the mating thread.

9. Clamping device (100) according to claim 8, **characterized by** the rotary joint (60) which comprises at least two rotary joint components (61, 62) mounted rotatably relative to one another about the axis of rotation (D) and adjoining one another, which can be locked relative to one another in different angular positions by means of the traction element (31), wherein a first further spring means (63) is arranged between the rotary joint components (61, 62), which is designed to bias the rotary joint components (61, 62) away from one another in the axial direction (A), wherein the rotary joint components (61, 62) have fourth surface structurings (64) arranged opposite one another and formed complementarily to one another, which are designed to engage into one another in a form-fitting manner in different angular positions , wherein the first further spring means (63) is preferably formed integrally with one of the rotary joint components (61, 62), wherein the first further spring means (63) particularly preferably protrudes from the rotary joint component (61, 62) in the axial direction (A).

10. Clamping device (100) according to one of claims 8 to 9, **characterized by** the swivel joint (70) which comprises at least two swivel joint components (71, 72) mounted pivotably relative to one another about the pivot axis (S) and adjoining one another, which can be locked relative to one another in different angular positions by means of the traction element (31), wherein a second further spring means (73) is arranged between the swivel joint components (71, 72), which is designed to bias the swivel joint components (71, 72) away from one another in the axial direction (A), wherein the swivel joint components (71, 72) have fifth surface structurings (74) arranged opposite one another and formed complementarily to one another, which are designed to engage into one another in a form-fitting manner in different angular positions.

11. Clamping device (100) according to one of the preceding claims,
**characterized by** the traction element (31) which is movably guided at the end in a ball joint of the second clamp (20), in particular of the fourth clamping jaw (22), wherein the traction element (31) has a head end (32) formed spherically at least in regions, wherein the head end (32) is movably guided in a joint socket (34) of the swivel joint (70).

12. Clamping device (100) according to claim 11, **characterized by** the head end (32) of the traction element (31) which has at least one fixing groove (35), into which a projection protruding from the joint socket (34) engages in such a way that a rotation of the traction element (31) about the axial direction (A) is at least restricted.

## Revendications

1. Dispositif de serrage (100) pour relier des broches (201) et/ou des éléments de barre (202) d'un fixateur externe (200) pour le traitement de fractures osseuses, présentant une première pince (10) et une deuxième pince (20), dans lequel un élément de traction (31) reliant la première pince (10) à la deuxième pince (20) est prévu le long d'une direction axiale (A) dans un alésage traversant (30) de la première pince (10) et de la deuxième pince (20) , dans lequel l'élément de traction (31) est monté par une première extrémité (31') dans la deuxième pince (20) et présente à une deuxième extrémité (31"), opposée à la première extrémité (31'), un filetage (33) qui s'engage dans un contre-filetage d'un moyen de serrage (40) , dans lequel l'élément de traction (31) est prévu pour le serrage de la première pince (10) et de la deuxième pince (20) par vissage du filetage (33) dans le contre-filetage, dans lequel le dispositif de serrage (100) présente un moyen de ressort (50) pour la précontrainte de la première pince (10) et de la deuxième pince (20), **caractérisé en ce que** le moyen de serrage (40) est disposé entre le moyen de ressort (50) et l'élément de traction (31), dans lequel le dispositif de serrage (100) présente un volant à main (41), dans lequel le volant à main (41) est en prise avec le moyen de serrage (40) pour faire tourner le moyen de serrage (40), dans lequel le moyen de serrage (40) est disposé de manière adjacente au volant à main (41) le long de la direction axiale (A) , dans lequel le moyen de serrage (40) est disposé entre le volant à main (41) et la première pince (10), dans lequel le contre-filetage du moyen de serrage (40) est disposé dans un premier alésage supplémentaire (42) du moyen de serrage (40) aligné avec l'alésage traversant (30), dans lequel une partie d'engagement (43) du moyen de serrage (40) est en prise par complémentarité de forme avec un logement (44) du volant à main (41) , dans lequel le volant à main (41) présente un évidement (45), dans lequel le moyen de ressort (50) est disposé à l'intérieur de l'évidement (45), dans lequel le moyen de ressort (50) est disposé en appui contre un point d'appui (46) du volant à main (41) et contre un moyen de précontrainte (51), dans lequel le moyen de précontrainte (51) est en prise avec l'élément de traction (31) , dans lequel le moyen de ressort (50) est disposé de telle sorte qu'une force de ressort du moyen de ressort (50) tire l'élément de traction (31) le long de la direction axiale (A) en direction du volant à main (41).

2. Dispositif de serrage (100) selon la revendication 1, **caractérisé en ce que** la première pince (10) présente une première mâchoire de serrage (11) et une deuxième mâchoire de serrage (12) et que la deuxième pince (20) présente une troisième mâchoire de serrage (21) et une quatrième mâchoire de serrage (22) , dans lequel la première mâchoire de serrage (11), la deuxième mâchoire de serrage (12), la troisième mâchoire de serrage (21) ainsi que la quatrième mâchoire de serrage (22) présentent chacune une rainure (13, 14, 23, 24), dans lequel une première rainure de réception ouverte (16) pour recevoir un élément de barre (202) est formée par les rainures (13, 14) de la première mâchoire de serrage (11) et de la deuxième mâchoire de serrage (12) et une deuxième rainure de réception ouverte (28) pour la réception d'un élément de barre (202) est formée par les rainures (22, 24) de la troisième mâchoire de serrage (21) et de la quatrième mâchoire de serrage (22) , dans lequel la troisième mâchoire de serrage (21) et la quatrième mâchoire de serrage (22) présentent chacune une, de préférence deux autres rainures (25), dans lequel au moins une, de préférence deux, autres rainures de réception ouvertes (29) pour la réception d'une broche (201) sont formées par les autres rainures (25) de la troisième mâchoire de serrage (21) et de la quatrième mâchoire de serrage (22) , dans lequel la première rainure de réception (16) est pourvue au moins par sections de préférence de premières structures de surface (15) qui sont conçues pour s'engager par complémentarité de forme dans des premières structures de surface supplémentaires (204) de forme complémentaire d'un élément de barre (202) et/ou la deuxième rainure de réception (28) est pourvue au moins par sections de préférence de deuxièmes structures de surface (26) qui sont conçues pour s'engager par complémentarité de forme dans des premières structures de surface supplémentaires (204) de forme complémentaire d'un élément de barre (202) et/ou l'autre rainure de réception (29) ou les autres rainures de réception (29) est/sont pourvue(s) au moins par sections de préférence de troisièmes structures de surface qui sont conçues pour s'engager par complémentarité de forme dans des deuxièmes structures de surface supplémentaires (203) de forme complémentaire d'une broche (201).

3. Dispositif de serrage (100) selon l'une des revendications précédentes, **caractérisé en ce que** l'évidement (45) est disposé de manière alignée avec l'alésage (30).

4. Dispositif de serrage (100) selon l'une des revendications précédentes, **caractérisé en ce que** le moyen de précontrainte (51) présente une vis de blocage dont le filetage supplémentaire (53) est en prise avec un contre-filetage supplémentaire (47) de l'élément de traction (31), dans lequel de préférence le moyen de ressort (50) est disposé en appui contre une face inférieure, tournée vers le point d'appui (46), d'une tête de vis (52) de la vis de blocage.

5. Dispositif de serrage (100) selon l'une des revendications précédentes, **caractérisé en ce que** le point d'appui (46) est disposé entre le logement (44) du volant à main (41) et l'évidement (45) du volant à main (41), dans lequel le point d'appui (46) est de préférence réalisé d'une seule pièce avec le volant à main (41) ou dans lequel le point d'appui (46) présente un disque annulaire qui est inséré dans une rainure annulaire du volant à main (41).

6. Dispositif de serrage (100) selon l'une des revendications précédentes, **caractérisé en ce que** le logement (44) présente une section transversale profilée, de préférence une section transversale ondulée et/ou angulaire, particulièrement de préférence carrée, hexagonale ou octogonale, dans lequel la partie d'engagement (43) est disposée sur une section supérieure (43') du moyen de serrage (40), dans lequel la section supérieure (43') présente une section transversale profilée, de préférence une section transversale ondulée et/ou angulaire, particulièrement de préférence carrée ou hexagonale.

7. Dispositif de serrage (100) selon la revendication 6, **caractérisé en ce que** le moyen de serrage présente une section inférieure (43"), dans lequel la section inférieure (43") est disposée dans l'alésage (30), dans lequel la section inférieure (43") présente de préférence une section transversale ronde.

8. Dispositif de serrage (100) selon l'une des revendications précédentes, **caractérisé en ce que** la première pince (10) et la deuxième pince (20) sont montées de manière rotative l'une par rapport à l'autre par l'intermédiaire d'une articulation rotative (60) autour d'un axe de rotation (D) et de manière pivotante l'une par rapport à l'autre par l'intermédiaire d'une articulation pivotante (70) autour d'un axe de pivotement (S) disposé perpendiculairement à l'axe de rotation (D), dans lequel l'élément de traction (31) est prévu pour fixer la première pince (10) par rapport à la deuxième pince (20) par rapport à l'axe de rotation (D) et à l'axe de pivotement (S) par vissage du filetage (33) dans le contre-filetage.

9. Dispositif de serrage (100) selon la revendication 8, **caractérisé en ce que** l'articulation rotative (60) comprend au moins deux composants d'articulation rotative (61, 62) adjacents et montés de manière rotative l'un par rapport à l'autre autour de l'axe de rotation (D), qui peuvent être bloqués l'un par rapport à l'autre dans différentes positions angulaires au moyen de l'élément de traction (31), dans lequel un premier moyen de ressort supplémentaire (63) est disposé entre les composants d'articulation rotative (61, 62), lequel est conçu pour écarter les composants d'articulation rotative (61, 62) l'un de l'autre dans la direction axiale (A), dans lequel les composants d'articulation rotative (61, 62) présentent des quatrièmes structures de surface (64) disposées de manière opposée l'une à l'autre et réalisées de manière complémentaire l'une par rapport à l'autre, qui sont conçues pour s'engager les unes dans les autres par complémentarité de forme dans différentes positions angulaires , dans lequel le premier moyen de ressort supplémentaire (63) est de préférence réalisé d'une seule pièce avec l'un des composants d'articulation rotative (61, 62), dans lequel le premier moyen de ressort supplémentaire (63) fait saillie particulièrement de préférence hors du composant d'articulation rotative (61, 62) dans la direction axiale (A).

10. Dispositif de serrage (100) selon l'une des revendications 8 à 9, **caractérisé en ce que** l'articulation pivotante (70) comprend au moins deux composants d'articulation pivotante (71, 72) adjacents et montés de manière pivotante l'un par rapport à l'autre autour de l'axe de pivotement (S), qui peuvent être bloqués l'un par rapport à l'autre dans différentes positions angulaires au moyen de l'élément de traction (31), dans lequel un deuxième moyen de ressort supplémentaire (73) est disposé entre les composants d'articulation pivotante (71, 72), lequel est conçu pour écarter les composants d'articulation pivotante (71, 72) l'un de l'autre dans la direction axiale (A), dans lequel les composants d'articulation pivotante (71, 72) présentent des cinquièmes structures de surface (74) disposées de manière opposée l'une à l'autre et réalisées de manière complémentaire l'une par rapport à l'autre, qui sont conçues pour s'engager les unes dans les autres par complémentarité de forme dans différentes positions angulaires.

11. Dispositif de serrage (100) selon l'une des revendications précédentes, **caractérisé en ce que** l'élément de traction (31) est guidé de manière mobile à l'extrémité dans un joint à rotule de la deuxième pince (20), en particulier de la quatrième mâchoire de serrage (22), dans lequel l'élément de traction (31) présente une extrémité de tête (32) réalisée de forme sphérique au moins par endroits, dans lequel l'extrémité de tête (32) est guidée de manière mobile dans une cavité d'articulation (34) de l'articulation pivotante (70).

12. Dispositif de serrage (100) selon la revendication 11, **caractérisé en ce que** l'extrémité de tête (32) de l'élément de traction (31) présente au moins une rainure de fixation (35) dans laquelle une saillie dépassant de la cavité d'articulation (34) s'engage de telle sorte qu'une rotation de l'élément de traction (31) autour de la direction axiale (A) est au moins limitée.
